(19)

Europäisches
Patentamt
European
Patent Office
Office européen
des brevets

(11) **EP 3 545 822 A1**

(12) **EUROPEAN PATENT APPLICATION**

(43) Date of publication:
**02.10.2019 Bulletin 2019/40**

(51) Int Cl.:
*A61B 5/00* (2006.01)          *A61B 5/024* (2006.01)
*A61B 5/1455* (2006.01)

(21) Application number: **18164185.3**

(22) Date of filing: **27.03.2018**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AE Eindhoven (NL)**

(72) Inventors:
• **CIUHU, Calina
5656 AE Eindhoven (NL)**

• **SHAN, Caifeng
5656 AE Eindhoven (NL)**
• **DE HAAN, Gerard
5656 AE Eindhoven (NL)**
• **Lai, Marco
5656 AE Eindhoven (NL)**

(74) Representative: **de Haan, Poul Erik et al
Philips International B.V.
Philips Intellectual Property & Standards
High Tech Campus 5
5656 AE Eindhoven (NL)**

(54) **DEVICE, SYSTEM AND METHOD FOR DETERMINING AT LEAST ONE VITAL SIGN OF A SUBJECT**

(57)     The present invention relates to a device, system and method for determining at least one vital sign of a subject. To solve the motion robustness problem of known solutions, the device comprises a receiver (210) configured to receive electromagnetic radiation reflected from a skin region of a subject, a polarization unit (220) configured to apply a first polarization on the received electromagnetic radiation to generate first polarized radiation and to apply a second polarization, which is different from the first polarization, on the received electromagnetic radiation to generate second polarized radiation, a sensor unit (230) configured to generate a first detection signal having a first polarization direction from the first polarized radiation and a second detection signal having a second polarization direction, which is different from the first polarization direction, from the second polarized radiation, and a vital sign determination unit (240) configured to determine a vital sign from the two detection signals by combining the two detection signals.

FIG.2

EP 3 545 822 A1

**Description**

FIELD OF THE INVENTION

The present invention relates to a device, system and method for determining at least one vital sign of a subject. Further, the present invention relates to a device for obtaining detection signals allowing to extract physiological information indicative of at least one vital sign of a subject.

BACKGROUND OF THE INVENTION

Vital signs of a person, for example the heart rate (HR), the respiration rate (RR) or the (peripheral or pulsatile) blood oxygen saturation (SpO2; it provides an estimate of the arterial blood oxygen saturation SaO2), serve as indicators of the current state of a person and as powerful predictors of serious medical events. For this reason, vital signs are extensively monitored in inpatient and outpatient care settings, at home or in further health, leisure and fitness settings.

One way of measuring vital signs is plethysmography. Plethysmography generally refers to the measurement of volume changes of an organ or a body part and in particular to the detection of volume changes due to a cardio-vascular pulse wave traveling through the body of a subject with every heartbeat.

Photoplethysmography (PPG) is an optical measurement technique that evaluates a time-variant change of light reflectance or transmission of an area or volume of interest. PPG is based on the principle that the blood absorbs light more than the surrounding tissue, so variations in blood volume with every heartbeat affect the transmission or reflectance correspondingly. Besides information about the pulse rate (heart rate), a PPG waveform (also called PPG signal) can comprise information attributable to further physiological phenomena such as the respiration. By evaluating the transmittance and/or reflectance at different wavelengths (typically red and infrared), the blood oxygen saturation can be determined.

Conventional pulse oximeters (also called contact PPG device herein) for measuring the pulse rate and the (arterial) blood oxygen saturation of a subject are attached to the skin of the subject, for instance to a fingertip, earlobe or forehead. Therefore, they are referred to as 'contact' PPG devices. Although contact PPG is basically regarded as a non-invasive technique, contact PPG measurement is often experienced as being unpleasant and obtrusive, since the pulse oximeter is directly attached to the subject and the cables limit the freedom to move and might hinder a workflow.

Non-contact, remote PPG (rPPG) devices (also called camera-based devices or video health monitoring devices) for unobtrusive measurements have been proposed in the last decade. Remote PPG utilizes light sources or, in general, radiation sources, disposed at a distance from the subject of interest. Similarly, a detector, e.g. a camera or a photodetector, can be disposed at a distance from the subject of interest. Therefore, remote photoplethysmographic systems and devices are considered unobtrusive and well suited for medical as well as non-medical everyday applications.

Using the PPG technology, vital signs can be measured. Vital signs are revealed by minute light absorption changes in the skin caused by the pulsating blood volume, i.e. by periodic color changes of the human skin induced by the blood volume pulse. As this signal is very small and hidden in much larger variations due to illumination changes and motion, there is a general interest in improving the fundamentally low signal-to-noise ratio (SNR). There still are demanding situations, with severe motion, challenging environmental illumination conditions, or strict accuracy requirements, where an improved robustness and accuracy of the vital sign measurement devices and methods is required, particularly for the more critical healthcare applications.

Video Health Monitoring (to monitor or detect e.g. heart rate, respiration rate, SpO2, actigraphy, delirium, etc.) is a promising emerging field. Its inherent unobtrusiveness has distinct advantages for patients with fragile skin, or in need of long-term vital signs monitoring, such as NICU patients, patients with extensive bums, mentally-ill patients that remove contact-sensors, or COPD patients who have to be monitored at home during sleep. In other settings such as in a general ward or emergency room, the comfort of contactless monitoring is still an attractive feature.

Hence, while being a promising new field, many challenges have to be overcome. Designing the system to be robust to movements of the patient is currently one of the main challenges. One of these challenges is to achieve motion robust SPO2 measurement with a vital signs camera as well as to obtain an improved motion robust pulse- and respiratory-signal, an improved pulse- and respiratory-rate, and e.g. a robust CO-level and an improved serum bilirubin estimation (i.e. essentially all PPG-based information shall be made more robust).

Motion robustness can be enabled by the fact that subject motion modulates the reflected light differently in different wavelength channels than the blood volume variations in the skin. Typically two types of motion-induced variations in the video signal can be observed:

-    Distance variations (square-law) change the intensity of the illumination, and so does the orientation of the skin surface with respect to the light source. The intensity of the reflected light changes, i.e. the color remains the same. In other words: the relative change is identical in all wavelength channels.

- Since skin is not a Lambertian reflector, but the reflected light also contains some portion of specularly reflected light (color of the light source, not of the skin), variations in the orientation of the skin surface relative to the light source also modulate the wavelength channels in another characteristic (pseudo-) color direction, related to the skin color.

These two distinct motion-induced distortions in the PPG signal from a camera can be suppressed using a linear de-mixing strategy, which however requires that at least three wavelength channels have to be available.

As there may be more distortion directions in a practical situation, e.g. due to multiple colored light sources, or colored reflections from nearby objects, a three wavelength system is not perfect, but usually suffices to obtain a PPG signal with a reasonable motion robustness.

In conclusion, there is a need for an improved device, system and method for determining at least one vital sign of a subject to obtain results with higher reliability even in case of noise and/or motion.

SUMMARY OF THE INVENTION

It is an object of the present invention to provide a device, system and method for extracting physiological information indicative of at least one vital sign of a subject, by which results with higher reliability, even in case of noise and/or motion, can be achieved.

In a first aspect of the present invention a device is presented comprising:

- a receiver configured to receive electromagnetic radiation reflected from a skin region of a subject,
- a polarization unit configured to apply a first polarization on the received electromagnetic radiation to generate first polarized radiation and to apply a second polarization, which is different from the first polarization, on the received electromagnetic radiation to generate second polarized radiation,
- a sensor unit configured to generate a first detection signal having a first polarization direction from the first polarized radiation and a second detection signal having a second polarization direction, which is different from the first polarization direction, from the second polarized radiation,

a vital sign determination unit configured to determine a vital sign from the two detection signals by combining the two detection signals.

In a further aspect of the present invention a system is presented comprising:

- an illumination unit configured to illuminate the skin region of the subject with unpolarized electromagnetic radiation or with linearly polarized electromagnetic radiation, and
- a device as disclosed herein for determining at least one vital sign of a subject from the received electromagnetic radiation.

In yet a further aspect of the present invention, there is provided a corresponding method.

Preferred embodiments of the invention are defined in the dependent claims. It shall be understood that the claimed method and system have similar and/or identical preferred embodiments as the claimed device, in particular as defined in the dependent claims and as disclosed herein.

In some application fields, only a single wavelength illumination is available, for instance in certain applications for automotive, where a PPG signal is measured remotely by only using the already available NIR illumination, which originates from a single LED light source (often emitting radiation around 850 nm). A camera registering the light reflected from the driver (e.g. the face) cannot distinguish between modulations caused by motion and modulations due to absorption changes of the skin caused by changing blood volume. Outside the automotive field, a single wavelength technology could become equally interesting for patient monitoring in hospitals. A (pseudo)-single wavelength technique is used for NIR remote PPG, which profits from the spreading of the emitted spectrum of the LEDs commonly used. Drawback of that approach is that the different wavelengths obtained from such a system are typically quite close to each other, and consequently their signal will exhibit a rather strong correlation. Although de-mixing is possible, because of the relatively high correlation between the channels, the signal-to-noise-ratio obtained is less than possible with more widely separated wavelength channels.

A problem addressed by the present invention is to obtain multiple channels with low correlation, suitable for motion-robustness improvement, while only using a single wavelength channel. This problem is solved by using two different polarizations (e.g. by use of two orthogonally oriented polarizers in front of two image sensors) to obtain fully independent channels at a single wavelength. Although this only provides two channels instead of the required three to deal with both intensity variations and specular reflection variations, it does allow complete elimination of the strongest distortion.

If polarized light is used, as provided in an embodiment, a cross-polarizer in front of a first image sensor can suppress the specular reflection (DC and variations) significantly, leaving the PPG signal and the intensity variations. The other sensor, equipped with a parallel polarizer, is then modulated by the PPG signal, the specular and the intensity variations. If the specular reflection variations are not the strongest distortion, the intensity variations artifact can be suppressed by mixing both channels. If specular distortion is strongest, only the cross-polarized channel may be used, while a small fraction of the parallel channel may be subtracted to compensate for imperfections of the polarizers. Further, methods that fully automatically decide upon the optimal de-mixing of the PPG signal may be used.

According to an embodiment said polarization unit is configured to apply the two different polarizations simultaneously and said sensor unit is configured to generate the two detection signals simultaneously. This embodiment may be implemented by used of a prism that that splits the incoming radiation into to output portions of radiation having different polarization directions.

Alternatively, said polarization unit may be configured to apply the two different polarizations time-sequentially and said sensor unit may be configured to generate the two detection signals time-sequentially. Hereby, said polarization unit may be configured to alternate the polarization direction in time. This embodiment may be implemented by use of a polarizer that is able to change the polarization like an electrically controllable polarization filter.

Generally, as polarizer a polarization filter may be used. However, for this purpose, not only transmission filters can be employed, but reflectors and/or mirrors (e.g. polarization mirrors) may be used to achieve the same effect.

Good results are achieved by use of a polarization unit that is configured to apply a first polarization which is orthogonal to the second polarization.

In another embodiment polarized illumination is used. Hereby, said polarization unit is configured to apply a first polarization which is parallel to the polarization direction of polarized electromagnetic radiation used for illuminating the skin region of the subject and a second polarization which is orthogonal to the polarization direction of the polarized electromagnetic radiation used for illuminating the skin region of the subject.

The sensor unit may be configured to generate detection signals in a single wavelength channel or in two or more different wavelength channels. For instance, a monochromatic sensor may be applied, or an RGB sensor (like a conventional image sensor) may be applied that provides three detection signal in three different color channels.

In an embodiment said vital sign determination unit is configured to determine a vital sign from the two detection signals by linearly combining the two detection signals by a weighted combination, wherein weights of said weighted combination are determined by blind signal separation, in particular by principal component analysis or independent component analysis, and by selecting a component channel of the combined detection signals according to a predetermined criterion. Such a blind signal separation method is e.g. described in WO 2017/121834 A1. The criterion may e.g. be the signal with highest peak in the normalized corresponding spectrum, or the signal with the maximum skewness of the corresponding spectrum, etc.

In another embodiment said vital sign determination unit is configured to determine a vital sign from the two detection signals by linearly combining the two detection signals by a weighted combination using weights resulting in a pulse signal for which the products with the original detection signals equals the relative pulsatilities as represented by the respective signature vector, a signature vector providing an expected relative strength of the detection signal in the two original detection signals.

Generally, a PPG signal results from variations of the blood volume in the skin. Hence, the variations give a characteristic pulsatility "signature" when viewed in different spectral components of the reflected/transmitted light. This "signature" is basically resulting as the contrast (difference) of the absorption spectra of the blood and that of the bloodless skin tissue. If the detector, e.g. a camera or sensor, has a discrete number of color channels, each sensing a particular part of the light spectrum, then the relative pulsatilities in these channels can be arranged in a "signature vector", also referred to as the "normalized blood-volume vector", PBV. It has been shown in G. de Haan and A. van Leest, "Improved motion robustness of remote-PPG by using the blood volume pulse signature", Physiol. Meas. 35 1913, 2014, which is herein incorporated by reference, that, if this signature vector is known, then a motion-robust pulse signal extraction on the basis of the color channels and the signature vector is possible. For the quality of the pulse signal, it is essential though that the signature vector is accurate, as otherwise the known methods mixes noise into the output pulse signal in order to achieve the prescribed correlation of the pulse vector with the normalized color channels as indicated by the signature vector.

Details of the PBV method and the use of the normalized blood volume vector (called "predetermined index element having a set orientation indicative of a reference physiological information") have also been described in US 2013/271591 A1, whose details are also herein incorporated by reference.

The characteristic wavelength-dependency of the PPG signal varies when the composition of the blood changes. Particularly, the oxygen saturation of the arterial blood has a strong effect on the light absorption in the wavelength range between 620 nm and 780 nm. This changing signature for different SpO2 values leads to relative PPG pulsatility that depends on the arterial blood oxygen saturation. This dependency can be used to realize a motion-robust remote SpO2 monitoring system that has been named adaptive PBV method (APBV) and is described in detail in M. van Gastel, S.

Stuijk and G. de Haan, "New principle for measuring arterial blood oxygenation, enabling motion-robust remote monitoring", Nature Scientific Reports, Nov. 2016. The description of the details of the APBV method in this document is also herein incorporated by reference.

The PBV method gives the cleanest pulse signal when the PBV vector, reflecting the relative pulsatilities in the different wavelength channels is accurate. Since this vector depends on the actual SpO2 value, testing the PBV method with different PBV vectors, corresponding to a range of SpO2 values, the SpO2 value results as the one corresponding to the PBV vector giving the pulse-signal with the highest SNR.

According to another aspect the present invention presents a system for determining at least one vital sign of a subject, said system comprising an illumination unit configured to illuminate the skin region of the subject with unpolarized electromagnetic radiation or with linearly polarized electromagnetic radiation and a device as described above for determining at least one vital sign of a subject from the received electromagnetic radiation.

The receiver of the proposed system may be configured in different ways, in particular to receive detection signals at different wavelengths, preferably depending on the kind of application and the system configuration. In general, the detection signals are selected from a wavelength interval between 300 nm and 1000 nm, in particular represent the wavelength portions corresponding to red, green and blue light. This is particularly used when the PPG signals are obtained from image signals acquired by a (e.g. conventional) video camera and when the above mentioned principles of remote PPG are used for deriving one or more vital signs. In other embodiments infrared light may also be used in addition or instead of another color channel. For instance, for night-time applications one or more infrared wavelengths may be used in addition or alternatively.

The receiver may be configured as optical element, e.g. a lens, of an imaging unit, such as an optical sensor, a camera, e.g. a video camera, an RGB camera or web-cam.

Preferably, the illumination unit is configured to illuminate the skin region of the subject with unpolarized electromagnetic radiation or with linearly polarized electromagnetic radiation within the wavelength range from 300 nm to 1000 nm.

According to an embodiment the illumination unit is configured to illuminate the skin region of the subject with linearly polarized electromagnetic radiation with a central wavelength in a wavelength range from 300 nm to 1000 nm, wherein the energy of the emitted electromagnetic radiation is spread in a wavelength interval around said central wavelength, wherein said sensor unit comprises two sensor elements, a first sensor element being configured to generate one or two first detection sub-signal having a polarization direction, which is parallel to the polarization direction of the polarized electromagnetic radiation, and a second sensor element being configured to generate one or two second detection sub-signal having a polarization direction, which is orthogonal to the polarization direction of the polarized electromagnetic radiation, wherein a first detection sub-signal represents electromagnetic radiation in a first wavelength sub-interval of said wavelength interval at least partly below said central wavelength and a second detection sub-signal represents electromagnetic radiation in a second wavelength sub-interval of said wavelength interval at least partly above said central wavelength, and wherein said vital sign determination unit is configured to determine a vital sign from the detection sub-signals by combining the detection sub-signals.

According to an alternative embodiment the illumination unit is configured to illuminate the skin region of the subject with linearly polarized electromagnetic radiation with a central wavelength in a wavelength range from 300 nm to 1000 nm, wherein the energy of the emitted electromagnetic radiation is spread in a wavelength interval around said central wavelength, wherein said sensor unit comprises four sensor elements, wherein a first and second sensor element are configured to generate one or two first detection sub-signal having a polarization direction, which is parallel to the polarization direction of the polarized electromagnetic radiation, and a third and fourth sensor element are configured to generate one or two second detection sub-signal having a polarization direction, which is orthogonal to the polarization direction of the polarized electromagnetic radiation, wherein one of the first and second detection sub-signals represents electromagnetic radiation in a first wavelength sub-interval of said wavelength interval at least partly below said central wavelength and the other of the first and second detection sub-signal represents electromagnetic radiation in a second wavelength sub-interval of said wavelength interval at least partly above said central wavelength, and wherein said vital sign determination unit is configured to determine a vital sign from the detection sub-signals by combining the detection sub-signals.

The first and second wavelength sub-intervals are generally different. For instance, the first wavelength sub-interval is below said central wavelength and the second wavelength sub-interval of said wavelength interval is above said central wavelength. Both wavelength sub-intervals may, however, also overlap.

The proposed system may further comprise an output unit configured to output the vital sign. The output unit may e.g. be a user interface like a display, computer or loudspeaker. Still further, the proposed system may comprise a control unit configured to generate, based on the vital sign, an alarm control signal for controlling an alarm unit configured to issue an alarm and to output the generated alarm control signal.

According to a further aspect, the present invention is directed to a device for obtaining detection signals allowing to extract physiological information indicative of at least one vital sign of a subject, said device comprising:

- a receiver configured to receive electromagnetic radiation reflected from a skin region of a subject,
- a polarization unit configured to apply a first polarization on the received electromagnetic radiation to generate first polarized radiation and to apply a second polarization, which is different from the first polarization, on the received electromagnetic radiation to generate second polarized radiation, and
- a sensor unit configured to generate a first detection signal having a first polarization direction from the first polarized radiation and a second detection signal having a second polarization direction, which is different from the first polarization direction, from the second polarized radiation.

BRIEF DESCRIPTION OF THE DRAWINGS

These and other aspects of the invention will be apparent from and elucidated with reference to the embodiment(s) described hereinafter. In the following drawings

Fig. 1 shows a schematic diagram of an embodiment of a system according to the present invention;
Fig. 2 shows a schematic diagram of an embodiment of a device for determining at least one vital sign of a subject according to the present invention; and
Fig. 3 shows a schematic diagram of an embodiment of a device for obtaining detection signals according to the present invention.

DETAILED DESCRIPTION OF THE INVENTION

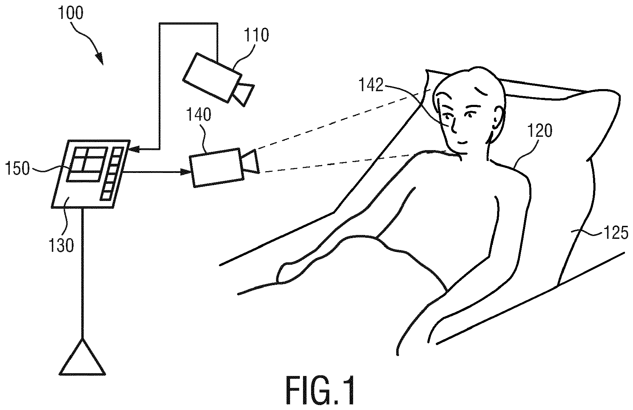

Fig. 1 shows a schematic diagram of an embodiment of a system 100 according to the present invention. The system 100 comprises an imaging unit 110 for receiving electromagnetic radiation reflected from a skin region of a subject 120. The system 100 further comprises a device 130 for determining at least one vital sign of a subject from the received electromagnetic radiation. The subject 120, in this example a patient, lies in a bed 125, e.g. in a hospital or other healthcare facility, but may also be a neonate or premature infant, e.g. lying in an incubator, or person at home or in a different environment.

The system 100 may further comprise a light source 140 (also called illumination unit), such as a lamp, for illuminating a region of interest 142 with linearly polarized electromagnetic radiation (light), such as the skin of the patient's face (e.g. part of the cheek or forehead), with light, for instance in a predetermined wavelength range or ranges (e.g. in the red, green and/or infrared wavelength range(s)) and with a predetermined polarization having a predetermined polarization direction. The light reflected from said region of interest 142 in response to said illumination is received by receiver 110, e.g. the lens of a camera or other optics in front of a sensor. In another embodiment no dedicated light source is provided, but ambient light is used for illumination of the subject 120. From the reflected light, only light in a number of desired wavelength ranges (e.g. green and red or infrared light, or light in a sufficiently large wavelength range covering at least two wavelength channels) may be detected and/or evaluated.

The device 130 is further connected to an interface 150 for displaying the determined information and/or for providing medical personnel with an interface to change settings of the device 130, the receiver 110, the light source 140 and/or any other parameter of the system 100. Such an interface 150 may comprise different displays, buttons, touchscreens, keyboards or other human machine interface means.

A system 100 as illustrated in Fig. 1 may, e.g., be located in a hospital, healthcare facility, elderly care facility or the like. Apart from the monitoring of patients, the present invention may also be applied in other fields such as neonate monitoring, general surveillance applications, security monitoring or so-called live style environments, such as fitness equipment, a wearable, a handheld device like a smartphone, or the like. The uni- or bidirectional communication between the device 130 and the interface 150 may work via a wireless or wired communication interface. Other embodiments of the present invention may include an imaging unit, which comprises part or all of the device 130, which is not provided stand-alone, but integrated into the imaging unit 110, such as a camera.

Typically, the electromagnetic radiation is in the range of 400 nm to 1000 nm for pulse, respiration and blood oxygen saturation measurement, particularly in the range of 620 nm to 920 nm. This particular range is most suitable for SpO2 measurement and is attractive for unobtrusive monitoring during sleep (darkness), but if pulse or respiratory signals are required, the visible part of the spectrum may allow a higher quality (i.e. NIR is not necessarily the preferred option in all cases).The detection signals may be acquired by a photo-sensor (array) and/or using a video camera remotely sensing the subject's skin.

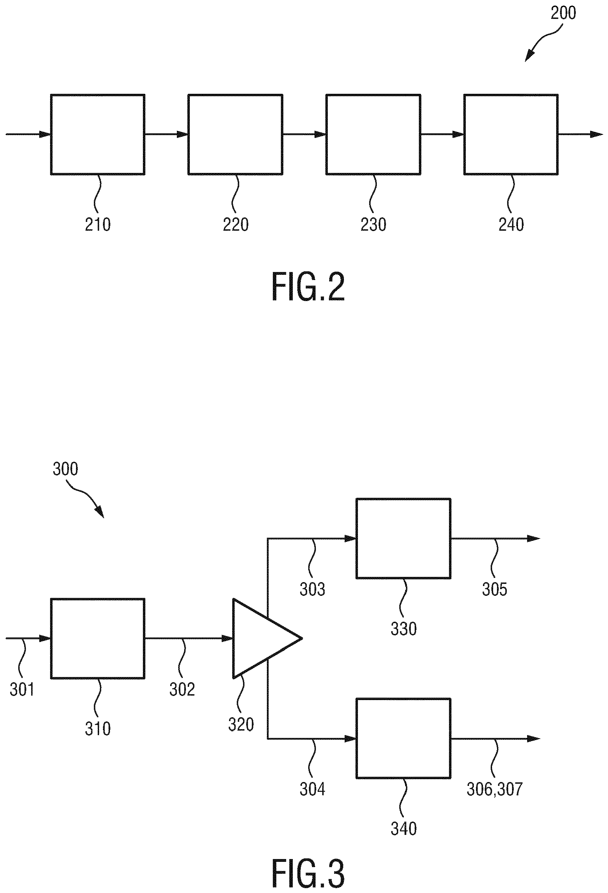

Fig. 2 shows a schematic diagram of a first embodiment of a device 200 according to the present invention for determining at least one vital sign of a subject. The device 200 may be integrated into the imaging unit 110, e.g. a camera, or may partly be integrated into or combined with the imaging unit 110 and partly be realized by the device 130, e.g. a processor or computer.

The device 200 comprises a receiver 210 configured to receive electromagnetic radiation reflected from a skin region of a subject. The receiver 210 may be an optical element, such as a lens of a camera that receives the radiation, in particular light in the desired wavelength range.

The device 200 further comprises a polarization unit 220 configured to apply a first polarization on the received electromagnetic radiation to generate first polarized radiation and to apply a second polarization, which is different from the first polarization, on the received electromagnetic radiation to generate second polarized radiation. The polarization unit 220 may e.g. comprise a prism or polarization filter(s). The first polarization may be parallel polarization and the second polarization may be cross polarization, which is orthogonal to the parallel polarization. However, other polarization directions and relationships of the polarizations are generally possible.

The device 200 further comprises a sensor unit 230 configured to generate a first detection signal having a first polarization direction from the first polarized radiation and a second detection signal having a second polarization direction, which is different from the first polarization direction, from the second polarized radiation. The sensor unit may comprise two separate sensor elements, each generating one of the two detection signals. The sensor unit (or each sensor element) may comprises a filter unit configured to filter the differently polarized radiation to obtain detection signals in two or more wavelength channels.

The device 200 further comprises a vital sign determination unit 240 configured to determine a vital sign from the two detection signals by combining the two detection signals. The vital sign determination unit 240 may be comprised in a digital or analog processor or computer and/or may completely or partly be implemented in software and carried out on a personal computer. Some or all of the required functionality may also be implemented in hardware, e.g. in an application specific integrated circuit (ASIC) or in a field programmable gate array (FPGA). In an embodiment, vital sign determination unit 240 is part of the device 130 of the system 100. In another embodiment, vital sign determination unit 240 is integrated into the imaging unit 110.

The present invention exploits the observation that the light reflected from the skin has two components: the surface reflection component and the component that originates from scattering processes underneath the surface in the translucent skin tissue. The surface reflection undergoes single scattering event, preserving the polarization of the incident light. The scattered light, undergoes multiple reflections, causing the incident light to de-polarize. The amount of depolarization can be expressed as:

$$P = \frac{I_p - I_c}{I_p + I_c} \tag{1}$$

where $I_p$, and $I_c$ are the intensity of the reflected light parallel (index p) and perpendicular (index c) to the incident light polarization. It is reasonable to assume that the original polarization of light is lost in biological perfused tissue, although the present invention works as well if partial polarization still exists.

Only the scattered component is actually modulated by the blood-volume changes that occur inside the tissue, while the surface (or specular) reflection component remains unmodulated. Therefore the pulsatility modulated signal can be separated from the non-modulated signal by using light of a determined polarization to illuminate the subject. In other words, with polarized light, only the scattered reflections become visible when using orthogonal (cross) polarizer in front of the camera. With parallel oriented polarizer in front of the camera, the surface reflection dominates the image.

For the remote PPG, since the scattered component contains the information about the variations of the blood volume, only the scattered light may be considered to extract the PPG signal. As the reflected photons that have penetrated into the skin have been scattered multiple times inside the skin, they have lost their initial polarization direction. This is in contrast with the specularly reflected light that keeps the polarization of the incident light. By using polarized illumination, a cross-polarizer in front of the imaging sensor suppresses specularly reflected light. Since the specular (surface) reflection is typically not desired, a sensor with a parallel polarizer has therefore not been used so far.

In an embodiment it is proposed to simultaneously, or time-sequentially, image with different polarizers, e.g. with both specular (parallel-polarized) and scattered (crossed-polarized) light. The resulting channels (different polarizations) lead to different mixtures of the wanted PPG signal and motion artefacts, which can be combined to extract a motion-robust PPG signal.

Fig. 3 shows a schematic diagram of a first embodiment of a device 300 for obtaining detection signals. Polarized illumination (polarized light) is emitted (by the illumination unit 140; see Fig. 1) to the skin of the subject. Light 301 reflected from the skin is received by the receiving element 310, e.g. an optical lens. The received light 302 is guided to an optical element 320, e.g. a polarization unit, that separates the cross and parallel polarized beams 303, 304 and projects them on separate sensors 330, 340 (together e.g. forming a sensor unit). In an exemplary embodiment the sensors 330, 340 are both equipped with a pixelated (Bayer) filter to acquire the different wavelength channel in parallel for each polarization direction. However, various alternatives exist.

In this embodiment, the polarization unit 320 (e.g. a prism or polarization filter) is generally configured to apply a first polarization on the received electromagnetic radiation 302 to generate first polarized radiation 303 and to apply a second polarization, which is different from the first polarization, or no polarization on the received electromagnetic radiation 302 to generate second polarized or non-polarized radiation 304. Further, the sensor unit comprising the sensors 330, 340 is generally configured to derive at least one detection signal 305 from the first polarized radiation 303 in a first wavelength channel and to derive at least two detection signals 306, 307 from the second polarized or non-polarized radiation 304 in the first wavelength channel and in a second wavelength channel.

In further embodiments it is provided that each sensor 330, 340 derives two or three detection signals in two or three wavelength channels, and or that the light beams 303, 304 are split further, separate wavelength selective filters are used in each path to a separate sensor.

With this setup, not only (half of) the scattered reflection can be selected while suppressing the specular reflection component using a cross-polarization direction, but the reflection can be captured as well with a parallel polarizer which fully passes the specular reflection and half of the scattered reflection (assuming photons have scattered so many times that strength of reflection is identical in all polarization directions, including cross and parallel directions).

A time-sequential setup may reduce the cost of the implementation significantly, although motion compensation is required to register the time-sequential image data (e.g. video images), which may introduce some weakness into the system for particular motions.

The acquired time signals from the two sensors 330, 340 are mixtures of motion artifacts and PPG signals, and different polarizations (angle between polarizers) can change the relative contribution of motion, leading to different mixtures. Having a (sufficiently high) number of independent mixtures allows extracting the wanted signal by linear de-mixing.

Only two independent channels are available in this setup, and for ease of discussion these shall be chosen as the cross and the parallel channels (all other polarizer directions are mixtures of these two). For this choice, the cross channel carries the PPG signal and (multiplicative) the intensity variations induced by motion, while the parallel channel carries an equally strong PPG signal (assuming this is equally spread over all polarization directions) plus the specular reflection variations, both multiplicatively affected by the intensity variations.

In general, for the skin-reflection model, without the use of polarizers, it holds:

$$C(t) = I(t).(v_s(t) + v_d(t) + v_n(t)) \qquad (2)$$

where $\mathbf{C}(t)$ denotes the camera output, $I(t)$ denotes the intensity level of the reflected light which, for a constant light-source, may be modulated by distance, or skin-surface orientation, variations due to subject-motion, while $\mathbf{C}(t)$ further depends on the time-varying (due to subject motion) specular reflection $v_s(t)$, and the time-varying (due to blood-volume variations) diffuse reflection component $v_d(t)$. The last term, $v_n(t)$, is a noise term, e.g. due to quantization and sensor noise.

Neglecting the noise term, the imaging sensor output can be separately written for the setup with cross and parallel polarizers under the assumption of polarized illumination and equal strength of the diffuse reflection in all polarization directions:

$$Cp(t) = I(t).(v_s(t) + v_d(t))$$
$$Cc(t) = I(t).v_d(t) \qquad (3)$$

If the above channels are divided by the DC value of the cross-polarized channel, $C_c(t)$, it can be written in approximation (assuming AC variations to be small compared to DC, and the DC specular reflection small compared to the diffuse reflection):

$$Cp(t) = 1 + I_{ac}(t) + v_s(t) + PPG(t)$$
$$Cc(t) = 1 + I_{ac}(t) + PPG(t) \qquad (4)$$

where $I_{ac}(t)$ is the AC part of I only, and PPG(t) is the AC part of the diffuse reflection term $v_d(t)$.

As can be seen from Eq. (4), there are only two equations and three unknowns. However, it can be solved for the wanted signal PPG(t), if the motion-induced variations (AC) in the intensity are small. With ideal polarizers, it would suffice to just use the signal from the camera with cross-polarized light. With non-ideal polarizers the signals from the two sensors are different mixtures of cross- and parallel-polarized signals. Hence, a linear combination of the two can suppress the cross-talk caused by imperfect polarizers, according to the present invention.

In an alternative embodiment, where the AC variations in $V_s$ are small, but the intensity variation, $I_{ac}$, not, a solution can now be provided. Starting from Eq. (3), both polarized channels are first normalized by dividing by their respective temporal mean, and the following approximation may be used:

$$Cp(t) = 1 + I_{ac}(t) + PPG(t)/(1 + v_s(t))$$
$$Cc(t) = 1 + I_{ac}(t) + PPG(t) \qquad (5)$$

Since the DC component of $V_s$ is positive and non-zero, the two channels provide different mixtures of $I_{ac}$ and the PPG signal, so it can be solved for PPG (limited SNR if DC part of specular is small).

In the final case where both $I_{ac}$ and the AC component of $V_s$ are relatively large compared to the PPG signal, it cannot be solved for PPG with only two equations, but instead a compromise can be made between suppression of both components, which may still be somewhat better than only suppressing one distortive component.

Rather than trying to strike a balance for optimal suppression of the two motion-induced distortion components, in an embodiment the described use of polarizers is combined with another idea. In particular, using cameras (in particular sensors) with pixelated optical filters (e.g. Bayer-like filters), (slightly) different wavelength interval channels can be obtained for each polarization direction, even for a single LED light source. This provides four channels with different mixtures of PPG signal and distortive components, and in total three distortions can be suppressed.

In such an embodiment the subject is e.g. irradiated with linearly polarized electromagnetic radiation with a light source (e.g. an LED) with a central wavelength (e.g. 550 nm, or 840 nm) in the range between 300 nm and 1000 nm, wherein the light source spreads the emitted energy in a small band (e.g. +/-100 nm) around the central wavelength. Three or four detection signals are obtained from two image sensors, one detecting parallel polarized radiation, and the other cross-polarized radiation. At least one of the sensors is equipped with a pixelated filter, such that some pixels reflect irradiation at the lower side of the central wavelength and other pixels reflect irradiation at the higher side of the central wavelength (the optical filter bands may overlap, which may allow for low-cost filters). In this case the vital sign determination unit computes the vital sign(s) by combining said three or four detection signals, e.g. by respectively subtracting the two sub-signals having the same polarization but reflecting the radiation from the two different wavelength intervals from each other. For instance, a first difference is determined between the first and second detection sub-signals of parallel polarization and a second difference is determined between the first and second detection sub-signals of cross-polarization, i.e. time-variant detection sub-signals are subtracted from each other (at each sampling time the values of the detection sub-signals are subtracted). Hereby, the detection sub-signals are preferably normalized first, i.e. by taking their logarithm, or by dividing the samples of each wavelength channel by their temporal mean.

In a further embodiment none of the sensors is equipped with a pixelated filter, but rather one or both polarized radiation paths are split into two beams directed at two sensors such that one sensor reflects irradiation at the lower side of the central wavelength and the other reflects irradiation at the higher side of the central wavelength.

In all above cases, Blind Source Separation (BSS) techniques, like Principle Component Analysis (PCA), or Independent Component Analysis (ICA) may be used to extract the independent signals, as e.g. described in the above cited paper of G. de Haan and A. van Leest, "Improved motion robustness of remote-PPG by using the blood volume pulse signature". PCA requires the relative energy of the different components to be sufficiently different, but is often simpler and more robust than ICA, which however can deal with independent signals with equal energy in the mix. Whichever BSS method is being used, two independent signals are obtained from the two sensor signals. To choose the PPG signal, a common strategy is to find the one that is most periodic. To decide this, often a Fourier transform on a time-lapse of the signals is done and the selection is based on this transform, e.g. by choosing the signal of which the spectrum has the lowest entropy, or the spectrum with the highest skewness, or the spectrum with the highest frequency peak after normalizing the spectrum.

Another preferred option is to use the PBV methods as e.g. described as well in the above cited paper of G. de Haan and A. van Leest, "Improved motion robustness of remote-PPG by using the blood volume pulse signature", to find the motion-robust PPG signal. To describe the procedure in this case, normalized signals are written from both sensors in a time window as vectors, Cp and Cc, that are combined into a matrix, Cn=[Cp, Cc], and the extracted pulse (PPG) signal is written as S, which is a weighted sum of the two sensor signals:

$$S = W\ C_n \qquad (6)$$

The PBV-method obtains the mixing coefficients using the prior knowledge regarding the relative strength of the pulse in the two camera-channels. In our case, we expect the pulse to be equally strong in both channels, i.e. Pbv= [1, 1], provided both channels are normalized by the DC value of the cross-polarized channel. The best results are obtained if the band-passed filtered versions of the two polarized channels are used.

According to this method the known $P_{bv}$ vector is used to discriminate between the pulse signal and distortions. Given that the relative amplitude of the pulse signal at in cross- and polarized channel is given by $P_{bv}$, the weights, $W_{PBV}$, are searched that give a pulse signal S, for which the correlation with the two polarized channels Cp and Cc equals $P_{bv}$

$$\vec{S}\mathrm{C}_\mathrm{n}^T = k\vec{P}_\mathrm{bv} \Leftrightarrow \vec{W}_\mathrm{PBV}\mathrm{C}_\mathrm{n}\mathrm{C}_\mathrm{n}^T = k\vec{P}_\mathrm{bv},$$

and consequently the weights determining the mixing are determined by

$$\vec{W}_\mathrm{PBV} = k\vec{P}_\mathrm{bv}\mathrm{Q}^{-1} \text{ with } \mathrm{Q} = \mathrm{C}_\mathrm{n}\mathrm{C}_\mathrm{n}^T,$$

and the scalar k can be determined such that $W_{PBV}$ has unit length (or another fixed length).

In other words, the weights indicate how the detection signals should be (linearly) combined in order to extract a pulse signal from the detection signals. The weights are unknown and need to be computed/selected.

The signature vector (PBV vector) represent the given (known or expected) relative pulsatilities in different wavelength channels (i.e. the detection signals), caused by the absorption spectrum of the blood and the penetration of light into the skin (if photons are more absorbed by blood, a volume change of blood leads to a larger signal than when the blood is nearly transparent). With this knowledge, and the observed data (i.e. the detection signals) the weights (e.g. a weight vector) can be determined. The resulting weights are data dependent, i.e. depend on the detection signals.

Since the pulse signal has a different ratio AC/DC (this is also called the relative signal strength / pulsatility) in each wavelength channel, it can be seen that the spectrum shows the pulse peak in the spectrum with different peak values for the different colors. This spectrum is the result of a Fourier analysis, but it basically means that if a sinusoid having the pulse frequency is correlated (multiplied) with the detection signals (RGB in the example, NIR-wavelengths for SpO2), exactly the peak values in the spectrum are obtained, which by definition are called the signature vector (PBV vector): these peak values are the relative strength of the normalized amplitudes of the pulse signal in the different detection signals.

The consequence of this is that a clean pulse signal S can be obtained (assuming the pulse signal is the result of a weighted sum of the detection signals), using this prior knowledge (i.e. the signature vector). One option to do this is to compute an inversion of a covariance matrix Q of the normalized detection signals $C_n$. Hence, the weights W to linearly mix the detection signals in order to extract the pulse signal S can be computed from the covariance matrix of the detection signals in the current analysis window (Q, which is data dependent, i.e. changes continuously over time), using the constant signature vector PBV.

In the above, it has been assumed that depolarization of the light scattered inside the skin is complete. This assumption makes it easy, since the PBV vector can be known a priory, but all above methods can also work if the assumption is not true. For the BSS methods, nothing changes, only for the PBV method a different vector may be used.

Finally, in all above embodiments, it has been assumed that the subject is illuminated with polarized light. However, even when unpolarized illumination is applied the system can work and the present invention can provide useful results, although it becomes a bit more complex. Essential here is the observation that the incident plane (incident light direction and the normal to the (skin)-surface) is the plane determining the partial polarization of the light. In applications when this plane is well defined (i.e. when scattering on a flat surface), well defined polarization on the lens with respect to the incidence plane should be sufficient to reduce the specular reflection and consequently cause a system with different polarizers to have different mixtures of the specular component. However, on most body parts, the skin is curved and the skin-normal varies spatially. Still, however, with local processing, a local demixing (same as above, but performed for individual patches of skin), solutions are assumed feasibly.

The above described methods can be applied on detection signals that have been acquired using contactless sensors. By way of example, the present invention can be applied in the field of healthcare, e.g. unobtrusive remote patient monitoring, general surveillances, security monitoring and so-called lifestyle environments, such as fitness equipment or the like. Applications may include monitoring of oxygen saturation (pulse oximetry), pulse rate, blood pressure, cardiac output, changes of blood perfusion, assessment of autonomic functions, respiration and detection of peripheral vascular diseases. The present invention can e.g. be used for rapid and reliable pulse detection of a critical patient, for instance during automated CPR (cardiopulmonary resuscitation). The system can be used for monitoring of vital signs of neonates with very sensitive skin e.g. in NICUs and for patients with damaged (e.g. burnt) skin, but may also be more convenient than contact sensors as used in the general ward, and offer better solutions for motion robustness.

In the following, some more details regarding equations (3) to (5) shall be provided.

Normalizing by the DC component of the cross-polarized channel $C_c(t)$ shall first be explained:

$$C_c(t) = I(t)v_d(t) = (I^{DC}(t) + I^{AC}(t))(v_d^{DC}(t) + v_d^{AC}(t))$$

$$= I^{DC}(t)v_d^{DC}(t) + I^{AC}(t)v_d^{DC}(t) + I^{DC}(t)v_d^{AC}(t) + I^{AC}(t)v_d^{AC}(t)$$

$$\cong I^{DC}(t)v_d^{DC}(t) + I^{AC}(t)v_d^{DC}(t) + I^{DC}(t)v_d^{AC}(t)$$

The DC component of the cross polarized channel $C_c(t)$ is $I^{DC}(t)v_d^{DC}(t)$. By normalizing, it is obtained

$$c_c(t) = 1 + \frac{I^{AC}(t)}{I^{DC}(t)} + \frac{v_d^{AC}(t)}{v_d^{DC}(t)}$$

$$c_c(t) = 1 + i_{AC}(t) + PPG(t)$$

where the following notations were used:

$$c_c(t) = C_c(t)/I^{DC}(t)v_d^{DC}(t)$$

indicating the cross polarized channel, normalized by its DC component.

$$PPG(t) = \frac{v_d^{AC}(t)}{v_d^{DC}(t)}$$

$$i_{AC}(t) = \frac{I^{AC}(t)}{I^{DC}(t)}$$

Normalizing the $C_p(t)$ by the DC component of the cross-polarized channel $C_c(t)$ shall now be explained:

$$C_p(t) = I(t)(v_s(t) + v_d(t))$$

$$= (I^{DC}(t) + I^{AC}(t))(v_s^{DC}(t) + v_s^{AC}(t) + v_d^{DC}(t) + v_d^{AC}(t))$$

$$= I^{DC}(t)v_s^{DC}(t) + I^{AC}(t)v_s^{DC}(t) + I^{DC}(t)v_s^{AC}(t) + I^{AC}(t)v_s^{AC}(t)$$

$$+ I^{DC}(t)v_d^{DC}(t) + I^{AC}(t)v_d^{DC}(t) + I^{DC}(t)v_d^{AC}(t) + I^{AC}(t)v_d^{AC}(t)$$

$$\cong I^{DC}(t)v_d^{DC}(t) + I^{AC}(t)v_d^{DC}(t) + I^{DC}(t)v_d^{AC}(t)$$

$$+ I^{DC}(t)v_s^{DC}(t) + I^{AC}(t)v_s^{DC}(t) + I^{DC}(t)v_s^{AC}(t)$$

where the second order in AC terms are neglected, as they are much smaller as compared to the DC terms.

The following approximations may be applied:

$$v_s^{DC}(t) \ll v_d^{DC}(t)$$

$$v_s^{AC}(t) \ll v_s^{DC}(t)$$

$$v_d^{AC}(t) \ll v_d^{DC}(t)$$

$$I^{AC}(t) \ll I^{DC}(t)$$

Normalizing the $C_p(t)$ by the DC component of the cross-polarized channel $C_c(t)$ will then result in:

$$C_p(t) = I(t)(v_s(t) + v_d(t))$$

$$= (I^{DC}(t) + I^{AC}(t))(v_s^{DC}(t) + v_s^{AC}(t) + v_d^{DC}(t) + v_d^{AC}(t))$$

$$= I^{DC}(t)v_s^{DC}(t) + I^{AC}(t)v_s^{DC}(t) + I^{DC}(t)v_s^{AC}(t) + I^{AC}(t)v_s^{AC}(t)$$

$$+ I^{DC}(t)v_d^{DC}(t) + I^{AC}(t)v_d^{DC}(t) + I^{DC}(t)v_d^{AC}(t) + I^{AC}(t)v_d^{AC}(t)$$

$$\cong I^{DC}(t)v_d^{DC}(t) + I^{AC}(t)v_d^{DC}(t) + I^{DC}(t)v_d^{AC}(t)$$

$$+ I^{DC}(t)v_s^{DC}(t) + I^{AC}(t)v_s^{DC}(t) + I^{DC}(t)v_s^{AC}(t)$$

$$\cong I^{DC}(t)v_d^{DC}(t) + I^{AC}(t)v_d^{DC}(t) + I^{DC}(t)v_d^{AC}(t)$$

$$+ I^{DC}(t)v_s^{DC}(t) + I^{DC}(t)v_s^{AC}(t)$$

$$\cong I^{DC}(t)v_d^{DC}(t) + I^{AC}(t)v_d^{DC}(t) + I^{DC}(t)v_d^{AC}(t)$$

$$+ I^{DC}(t)v_s(t)$$

The DC component of the cross polarized channel $C_c(t)$ is $I^{DC}(t)v_d^{DC}(t)$. By normalizing, it is obtained

$$c_p(t) = 1 + \frac{I^{AC}(t)}{I^{DC}(t)} + \frac{v_d^{AC}(t)}{v_d^{DC}(t)} + \frac{v_s(t)}{v_d^{DC}(t)}$$

$$c_p(t) = 1 + i_{AC}(t) + PPG(t) + \tilde{v}_s(t)$$

where the following notations were used:

$c_p(t) = C_p(t)/I^{DC}(t)v_d^{DC}(t)$ - the parallel polarized channel, normalized by the DC component of the crossed polarized channel

$$PPG(t) = \frac{v_d^{AC}(t)}{v_d^{DC}(t)}$$

$$i_{AC}(t) = \frac{I^{AC}(t)}{I^{DC}(t)}$$

$$\widetilde{v}_s(t) = \frac{v_s(t)}{v_d^{DC}(t)}$$

The AC variations in $v_s(t)$ are small, but $I^{AC}(t)$ are not both channels are normalized by dividing by their respective temporal mean $\langle C_p(t)\rangle$ and $\langle C_c(t)\rangle$ respectively

$$\left\langle C_p(t)\right\rangle = \langle I(t)(v_s(t))\rangle + \langle I(t)(v_d(t))\rangle$$

$$\langle C_c(t)\rangle = \langle I(t)(v_d(t))\rangle$$

resulting in

$$\left\langle C_p(t)\right\rangle = \langle I(t)(v_s(t))\rangle + \langle I(t)(v_d(t))\rangle$$
$$= I^{DC}(t)v_s(t) + I^{DC}(t)v_d^{DC}(t)$$
$$= I^{DC}(t)v_d^{DC}(t)\left(1 + \frac{v_s(t)}{v_d^{DC}(t)}\right)$$
$$= I^{DC}(t)v_d^{DC}(t)\left(1 + \widetilde{v}_s(t)\right)$$

$$\langle C_c(t)\rangle = \langle I(t)(v_d(t))\rangle = I^{DC}(t)v_d^{DC}(t)$$

Normalizing the $C_p(t)$ by the time-average component of the parallel-polarized channel $C_p(t)$ results in

$$C_p(t) = I(t)(v_s(t) + v_d(t))$$
$$= (I^{DC}(t) + I^{AC}(t))(v_s^{DC}(t) + v_s^{AC}(t) + v_d^{DC}(t) + v_d^{AC}(t))$$
$$= I^{DC}(t)v_s^{DC}(t) + I^{AC}(t)v_s^{DC}(t) + I^{DC}(t)v_s^{AC}(t) + I^{AC}(t)v_s^{AC}(t)$$
$$+I^{DC}(t)v_d^{DC}(t) + I^{AC}(t)v_d^{DC}(t) + I^{DC}(t)v_d^{AC}(t) + I^{AC}(t)v_d^{AC}(t)$$
$$\cong I^{DC}(t)v_d^{DC}(t) + I^{AC}(t)v_d^{DC}(t) + I^{DC}(t)v_d^{AC}(t)$$
$$+I^{DC}(t)v_s^{DC}(t) + I^{AC}(t)v_s^{DC}(t) + I^{DC}(t)v_s^{AC}(t)$$
$$\cong I^{DC}(t)v_d^{DC}(t) + I^{AC}(t)v_d^{DC}(t) + I^{DC}(t)v_d^{AC}(t)$$
$$+I^{DC}(t)v_s^{DC}(t) + I^{DC}(t)v_s^{AC}(t)$$
$$\cong I^{DC}(t)v_d^{DC}(t) + I^{AC}(t)v_d^{DC}(t) + I^{DC}(t)v_d^{AC}(t)$$
$$+I^{DC}(t)v_s(t)$$

The time average component of the parallel polarized channel $C_p(t)$ is $I^{DC}(t)v_d^{DC}(t)\left(1 + \widetilde{v}_s(t)\right)$ By normalizing, it is obtained

$$c_p(t) = \frac{1}{\left(1+\widetilde{v_s}(t)\right)} + \frac{I^{AC}(t)}{I^{DC}(t)\left(1+\widetilde{v_s}(t)\right)} + \frac{v_d^{AC}(t)}{v_d^{DC}(t)\left(1+\widetilde{v_s}(t)\right)} + \frac{v_s(t)}{v_d^{DC}(t)} \frac{1}{\left(1+\widetilde{v_s}(t)\right)}$$

$$c_p(t) = 1 + \frac{I^{AC}(t)}{I^{DC}(t)\left(1+\widetilde{v_s}(t)\right)} + \frac{v_d^{AC}(t)}{v_d^{DC}(t)\left(1+\widetilde{v_s}(t)\right)}$$

$$c_p(t) = 1 + \frac{i^{AC}(t)}{\left(1+\widetilde{v_s}(t)\right)} + \frac{PPG(t)}{\left(1+\widetilde{v_s}(t)\right)} \cong 1 + i^{AC}(t) + \frac{PPG(t)}{\left(1+\widetilde{v_s}(t)\right)}$$

A last step approximation is applied because the AC variations in $v_s(t)$ are small, but $I^{AC}(t)$ are not, where

$$PPG(t) = \frac{v_d^{AC}(t)}{v_d^{DC}(t)}$$

$$i_{AC}(t) = \frac{I^{AC}(t)}{I^{DC}(t)}$$

$$\widetilde{v_s}(t) = \frac{v_s(t)}{v_d^{DC}(t)}$$

The cross polarized component is derived as in the earlier embodiment (quite straightforward and

$$\langle C_c(t)\rangle = \langle I(t)(v_d(t))\rangle = I^{DC}(t)v_d^{DC}(t)).$$

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. Device for determining at least one vital sign of a subject, said device comprising:

- a receiver (210) configured to receive electromagnetic radiation reflected from a skin region of a subject,
- a polarization unit (220) configured to apply a first polarization on the received electromagnetic radiation to generate first polarized radiation and to apply a second polarization, which is different from the first polarization, on the received electromagnetic radiation to generate second polarized radiation,
- a sensor unit (230) configured to generate a first detection signal having a first polarization direction from the first polarized radiation and a second detection signal having a second polarization direction, which is different from the first polarization direction, from the second polarized radiation,

a vital sign determination unit (240) configured to determine a vital sign from the two detection signals by combining the two detection signals.

2. Device as claimed in claim 1,
wherein said polarization unit (220) is configured to apply the two different polarizations simultaneously and said sensor unit (230) is configured to generate the two detection signals simultaneously.

3. Device as claimed in claim 1,
wherein said polarization unit (220) is configured to apply the two different polarizations time-sequentially and said sensor unit (230) is configured to generate the two detection signals time-sequentially.

4. Device as claimed in claim 3,
wherein said polarization unit (220) is configured to alternate the polarization direction in time.

5. Device as claimed in claim 1,
wherein said polarization unit (220) is configured to apply a first polarization which is orthogonal to the second polarization.

6. Device as claimed in claim 1,
wherein said polarization unit (220) is configured to apply a first polarization which is parallel to the polarization direction of polarized electromagnetic radiation used for illuminating the skin region of the subject and a second polarization which is orthogonal to the polarization direction of the polarized electromagnetic radiation used for illuminating the skin region of the subject.

7. Device as claimed in claim 1,
wherein said sensor unit (230) is configured to generate detection signals in a single wavelength channel or in two or more different wavelength channels.

8. Device as claimed in claim 1,
wherein said vital sign determination unit (240) is configured to determine a vital sign from the two detection signals by linearly combining the two detection signals by a weighted combination, wherein weights of said weighted combination are determined by blind signal separation, in particular by principal component analysis or independent component analysis, and by selecting a component channel of the combined detection signals according to a predetermined criterion.

9. Device as claimed in claim 1,
wherein said vital sign determination unit (240) is configured to determine a vital sign from the two detection signals by linearly combining the two detection signals by a weighted combination using weights resulting in a pulse signal for which the products with the original detection signals equals the relative pulsatilities as represented by the respective signature vector, a signature vector providing an expected relative strength of the detection signal in the two original detection signals.

10. System for determining at least one vital sign of a subject, said system comprising:

- an illumination unit (140) configured to illuminate the skin region of the subject with unpolarized electromagnetic radiation or with linearly polarized electromagnetic radiation, and
- a device (200) as claimed in claim 1 for determining at least one vital sign of a subject from the received electromagnetic radiation.

11. System as claimed in claim 10,
wherein said illumination unit (140) is configured to illuminate the skin region of the subject with unpolarized electromagnetic radiation or with linearly polarized electromagnetic radiation within the wavelength range from 300 nm to 1000 nm.

12. System as claimed in claim 10,
wherein said illumination unit (140) is configured to illuminate the skin region of the subject with linearly polarized electromagnetic radiation with a central wavelength in a wavelength range from 300 nm to 1000 nm, wherein the energy of the emitted electromagnetic radiation is spread in a wavelength interval around said central wavelength,

wherein said sensor unit comprises two sensor elements (330, 340), a first sensor element being configured to generate one or two first detection sub-signal having a polarization direction, which is parallel to the polarization direction of the polarized electromagnetic radiation, and a second sensor element being configured to generate one or two second detection sub-signal having a polarization direction, which is orthogonal to the polarization direction of the polarized electromagnetic radiation, wherein a first detection sub-signal represents electromagnetic radiation in a first wavelength sub-interval of said wavelength interval at least partly below said central wavelength and a second detection sub-signal represents electromagnetic radiation in a second wavelength sub-interval of said wavelength interval at least partly above said central wavelength, and

wherein said vital sign determination unit (240) is configured to determine a vital sign from the detection sub-signals by combining the detection sub-signals.

13. System as claimed in claim 10,

wherein said illumination unit (140) is configured to illuminate the skin region of the subject with linearly polarized electromagnetic radiation with a central wavelength in a wavelength range from 300 nm to 1000 nm, wherein the energy of the emitted electromagnetic radiation is spread in a wavelength interval around said central wavelength, wherein said sensor unit (230) comprises four sensor elements, wherein a first and second sensor element are configured to generate one or two first detection sub-signal having a polarization direction, which is parallel to the polarization direction of the polarized electromagnetic radiation, and a third and fourth sensor element are configured to generate one or two second detection sub-signal having a polarization direction, which is orthogonal to the polarization direction of the polarized electromagnetic radiation,

wherein one of the first and second detection sub-signals represents electromagnetic radiation in a first wavelength sub-interval of said wavelength interval at least partly below said central wavelength and the other of the first and second detection sub-signal represents electromagnetic radiation in a second wavelength sub-interval of said wavelength interval at least partly above said central wavelength, and

wherein said vital sign determination unit (240) is configured to determine a vital sign from the detection sub-signals by combining the detection sub-signals.

14. Method for determining at least one vital sign of a subject, said method comprising:

- receiving electromagnetic radiation reflected from a skin region of a subject,
- applying a first polarization on the received electromagnetic radiation to generate first polarized radiation and a second polarization, which is different from the first polarization, on the received electromagnetic radiation to generate second polarized radiation,
- generating a first detection signal having a first polarization direction from the first polarized radiation and a second detection signal having a second polarization direction, which is different from the first polarization direction, from the second polarized radiation,

determining a vital sign from the two detection signals by combining the two detection signals.

15. Device for obtaining detection signals allowing to extract physiological information indicative of at least one vital sign of a subject, said device comprising:

- a receiver (310) configured to receive electromagnetic radiation reflected from a skin region of a subject,
- a polarization unit (320) configured to apply a first polarization on the received electromagnetic radiation to generate first polarized radiation and to apply a second polarization, which is different from the first polarization, on the received electromagnetic radiation to generate second polarized radiation, and
- a sensor unit (330, 340) configured to generate a first detection signal having a first polarization direction from the first polarized radiation and a second detection signal having a second polarization direction, which is different from the first polarization direction, from the second polarized radiation.

FIG.1

200

210    220    230    240

# FIG.2

300

301    310    302    320    303    330    305

304    340    306,307

# FIG.3

Europäisches
Patentamt

European
Patent Office

Office européen
des brevets

# EUROPEAN SEARCH REPORT

Application Number

EP 18 16 4185

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/120482 A1 (KIRENKO IHOR OLEHOVYCH [NL] ET AL) 5 May 2016 (2016-05-05) * paragraphs [0014], [0016], [0020], [0036], [0037], [0056]; figure 1 * | 1-15 | INV. A61B5/00 A61B5/024 A61B5/1455 |
| X | WO 2017/080869 A1 (KONINKLIJKE PHILIPS NV [NL]) 18 May 2017 (2017-05-18) * page 2, line 15 - line 17 * * page 4, line 14 - line 17 * * page 8, line 10 - line 11; figure 4 * * page 15, line 31 - page 16, line 3 * | 1-15 | |
| A,D | WO 2017/121834 A1 (KONINKLIJKE PHILIPS NV [NL]) 20 July 2017 (2017-07-20) * page 3, line 39 - line 30 * * page 5, line 16 - line 19 * * page 6, line 8 - line 19 * * claim 2 * | 1-15 | |
| A | US 2016/120468 A1 (MATHEW DENNY [NL] ET AL) 5 May 2016 (2016-05-05) * paragraph [0012]; figure 2 * | 1-15 | TECHNICAL FIELDS SEARCHED (IPC) A61B |

The present search report has been drawn up for all claims

| Place of search | Date of completion of the search | Examiner |
|---|---|---|
| The Hague | 9 August 2018 | Chau, Thoi Dai |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 18 16 4185

This annex lists the patent family members relating to the patent documents cited in the above-mentioned European search report.
The members are as contained in the European Patent Office EDP file on
The European Patent Office is in no way liable for these particulars which are merely given for the purpose of information.

09-08-2018

| Patent document cited in search report | | Publication date | Patent family member(s) | | Publication date |
|---|---|---|---|---|---|
| US 2016120482 | A1 | 05-05-2016 | CN | 107106017 A | 29-08-2017 |
| | | | EP | 3212059 A1 | 06-09-2017 |
| | | | JP | 2017532151 A | 02-11-2017 |
| | | | US | 2016120482 A1 | 05-05-2016 |
| | | | WO | 2016066724 A1 | 06-05-2016 |
| WO 2017080869 | A1 | 18-05-2017 | CN | 108289626 A | 17-07-2018 |
| | | | WO | 2017080869 A1 | 18-05-2017 |
| WO 2017121834 | A1 | 20-07-2017 | NONE | | |
| US 2016120468 | A1 | 05-05-2016 | CN | 107106051 A | 29-08-2017 |
| | | | EP | 3212060 A1 | 06-09-2017 |
| | | | US | 2016120468 A1 | 05-05-2016 |
| | | | WO | 2016066841 A1 | 06-05-2016 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017121834 A1

- US 2013271591 A1

**Non-patent literature cited in the description**

- **M. VAN GASTEL ; S. STUIJK ; G. DE HAAN.** New principle for measuring arterial blood oxygenation, enabling motion-robust remote monitoring. *Nature Scientific Reports,* November 2016